(19) **Europäisches Patentamt / European Patent Office / Office européen des brevets**

(11) **EP 1 618 864 B1**

(12) **FASCICULE DE BREVET EUROPEEN**

(45) Date de publication et mention
de la délivrance du brevet:
**13.04.2016 Bulletin 2016/15**

(51) Int Cl.:
*A61K 8/06* *(2006.01)*   *A61K 8/04* *(2006.01)*
*A61K 9/107* *(2006.01)*   *A61Q 19/00* *(2006.01)*
*A61Q 1/14* *(2006.01)*

(21) Numéro de dépôt: **05291243.3**

(22) Date de dépôt: **09.06.2005**

(54) **Emulsion H/E riche en huile, et son utilisation dans le domaine cosmétique**

Öl-in-Wasser-Emulsionen mit hohem Ölgehalt und deren Verwendung in der Kosmetik

High oil content oil-in-water emulsion and its use in cosmetics

(84) Etats contractants désignés:
**AT BE BG CH CY CZ DE DK EE ES FI FR GB GR HU IE IS IT LI LT LU MC NL PL PT RO SE SI SK TR**

(30) Priorité: **16.07.2004 FR 0451546**

(43) Date de publication de la demande:
**25.01.2006 Bulletin 2006/04**

(73) Titulaire: **L'Oréal**
**75008 Paris (FR)**

(72) Inventeurs:
• **Sebillotte-Arnaud, Laurence**
**94240 L'Hay les Roses (FR)**
• **Bordeaux, Dominique**
**94320 Thiais (FR)**

(74) Mandataire: **Duvert, Sandra**
**L'Oréal**
**D.I.P.I.**
**River PLaza**
**25-29 Quai Aulagnier**
**92600 Asnieres-sur-Seine (FR)**

(56) Documents cités:
**WO-A-01/89678    WO-A-98/09721**
**US-A- 4 606 913**

• **DATABASE CA CHEMICAL ABSTRACTS SERVICE, COLUMBUS, OHIO, US; AN: 137:52043 S. BABA: "Oily cosmetic compositions having improved storage stability and preservability" XP002270586**
• **ANONYMOUS: 'Ölbedarf', [en ligne] 17 Janvier 2012, pages 1 - 2 WIKIPEDIA Extrait de l'Internet: <URL:http://de.wikipedia.org/wiki/%C3%96lbe darf> [extrait le 2012-05-09]**
• **ANONYMOUS: 'Prüfmethoden für Farbmittel für Druckfarben', [en ligne] Octobre 2005, pages 1 - 8 BASF - The Chemical Company Extrait de l'Internet: <URL:http://www.dispersions-pigments.basf.c om/portal/streamer?fid=421489> [extrait le 2012-05-09]**
• **ANONYMOUS: 'ASTM D281 - 95(2007) Standard Test Method for Oil Absorption of Pigments by Spatula Rub-out', [en ligne] 2012, pages 1 - 2 ASTM International Extrait de l'Internet: <URL:http://www.astm.org/Standards/D281.htm > [extrait le 2012-05-09]**
• **ANONYMOUS: 'ASTM D1483 - 12 Standard Test Method for Oil Absorption of Pigments by Gardner-Coleman Method', [en ligne] 2012, pages 1 - 2 ASTM International Extrait de l'Internet: <URL:http://www.astm.org/Standards/D1483.ht m> [extrait le 2012-05-09]**
• **ANONYMOUS: 'Hydrophilic-lipophilic balance', [en ligne] 03 Avril 2012, pages 1 - 2 WIKIPEDIA Extrait de l'Internet: <URL:http://en.wikipedia.org/wiki/Hydrophil ic-lipophilic_balance> [extrait le 2012-05-09]**

EP 1 618 864 B1

**Description**

La présente invention se rapporte à une composition pour application topique, notamment cosmétique et/ou dermatologique, sous forme d'émulsion huile-dans-eau, riche en huile, susceptible d'être obtenue par inversion de phase, et à ses utilisations notamment dans le domaine cosmétique ou dermatologique, en particulier pour le traitement des matières kératiniques, le maquillage de la peau et pour le démaquillage de la peau, des muqueuses et/ou des cils.

Il est connu d'utiliser dans le domaine cosmétique ou dermatologique, des compositions huileuses, c'est-à-dire des compositions ne comprenant que des huiles ou contenant très peu d'eau, par exemple comme produits de démaquillage de la peau, comme produits de massage pour le soin du visage, du corps, du cuir chevelu ou des pieds, comme baume après-shampooing, comme huile de protection solaire ou encore comme huile de soin pour la douche. Ces compositions ont l'avantage d'être efficaces car les huiles se trouvent directement en contact avec la peau, mais elles présentent l'inconvénient d'être trop liquides, donc peu pratiques d'utilisation dans la mesure où elles coulent lors de l'application. De plus, elles ont l'inconvénient de manquer de fraîcheur et de laisser un effet gras lors de l'application sur la peau.

Les émulsions huile-dans-eau (H/E) sont constituées d'une phase huileuse (ou phase lipophile) dispersée dans une phase aqueuse. Elles ont donc une phase aqueuse externe et sont de ce fait des produits plus agréables à utiliser, en raison de la sensation de fraîcheur qu'elles procurent. Toutefois, elles présentent l'inconvénient de manquer relativement d'efficacité, notamment pour le démaquillage ou toute autre application où l'efficacité est d'autant plus grande que la quantité d'huile présente est plus importante, car la phase huileuse ou phase lipophile qui contient les huiles et qui constitue la phase interne n'est pas directement disponible et, de ce fait, l'efficacité de l'huile est moindre dans la mesure où la quantité d'huile est moins grande. Or, par exemple pour le nettoyage du visage et plus spécialement pour le démaquillage qui consiste à enlever tous les produits de maquillage, les femmes cherchent à obtenir un démaquillage le plus efficace possible.

Afin d'allier efficacité et agrément cosmétique, il est avantageux de réaliser une émulsion huile-dans-eau (H/E) contenant une quantité importante d'huile, et, pour faciliter la manipulation, il est avantageux que l'émulsion se présente sous forme de crème, ce qui permet de l'utiliser directement par préhension avec les doigts sans passer par un support tel qu'un coton, et sans craindre que le produit ne coule.

La fabrication d'émulsions H/E concentrées en huile est souvent difficile, et les émulsions obtenues sont souvent instables. Ces émulsions sont classiquement obtenues par voie mécanique, par exemple par émulsification avec un rotor stator ou à l'aide d'un homogénéisateur haute-pression, parce qu'il faut beaucoup d'énergie mécanique pour diviser la phase dispersée en petites gouttes. Pour stabiliser ces émulsions, on y ajoute généralement des tensioactifs émulsionnants du type huile-dans-eau c'est-à-dire de HLB (HLB = Hydrophilic Lipophilic Balance) allant de 8 à 18, émulsionnants qui, grâce à leur structure amphiphile, se placent à l'interface phase huileuse / phase aqueuse, et stabilisent ainsi les gouttelettes d'huiles dispersées. Malgré la présence d'émulsionnants, les émulsions peuvent avoir tendance à se déstabiliser (coalescence puis séparation des phases aqueuse et huileuse avec relargage d'huile). Pour améliorer la stabilité de ces émulsions, on peut augmenter la concentration en émulsionnants ; toutefois, une forte concentration en émulsionnants peut conduire à un toucher rêche, collant ou poisseux, ainsi qu'à des problèmes d'innocuité vis-à-vis de la peau, des yeux et du cuir chevelu.

Pour résoudre les problèmes de stabilité des émulsions H/E classiques, il a été proposé de réaliser des émulsions H/E obtenues par inversion de phase en température (émulsions PIT), dans lesquelles la taille moyenne des globules constituant la phase huileuse est comprise dans des limites bien déterminées, à savoir entre 0,1 à 4 $\mu$m (100 à 4000 nm). Le principe d'émulsification par inversion de phase en température (en Anglais : Phase Inversion Temperature ou PIT) est, dans son principe, bien connu de l'homme de l'art ; il a été décrit en 1968 par K. Shinoda (J. Chem. Soc. Jpn., 1968, 89, 435). Il a été montré que cette technique d'émulsification permet d'obtenir des émulsions fines stables (K. Shinoda et H. Saito, J. Colloïd Interface Sci., 1969,30, 258). Cette technologie a été appliquée en cosmétique dès 1972 par Mitsui et al. («Application of the phase-inversion-temperature method to the emulsification of cosmetics» ; T. Mitsui, Y. Machida and F. Harusawa, American. Cosmet. Perfum., 1972, 87, 33).

Le principe de cette technique est le suivant : on réalise le mélange d'une phase aqueuse et d'une phase huileuse, que l'on porte à une température supérieure à la température PIT, température d'inversion de phase du système, qui est la température à laquelle l'équilibre entre les propriétés hydrophile et lipophile du ou des émulsionnants mis en oeuvre est atteint ; à température élevée, c'est-à-dire supérieure à la température d'inversion de phase (>PIT), l'émulsion est de type eau-dans-huile, et, au cours de son refroidissement, cette émulsion s'inverse à la température d'inversion de phase, pour devenir une émulsion de type huile-dans-eau, et ceci en étant passée auparavant par un état de microémulsion. Ce procédé permet d'obtenir facilement des émulsions de diamètre inférieur à 4 $\mu$m.

Il est décrit dans le document WO-A-01/89678 des émulsions riches en huile, comportant un rapport en poids de la phase huileuse sur la phase aqueuse de 0,7. Toutefois, la vitesse de libération de l'huile de ces émulsions n'est pas suffisante pour obtenir une bonne efficacité, comme le montrent les exemples comparatifs présentés plus loin.

Il subsiste encore le besoin d'améliorer la vitesse de libération de l'huile des émulsions H/E fines, pour obtenir une meilleure efficacité, et donc de disposer d'émulsions H/E qui, bien que contenant une quantité importante d'huiles, soient stables, tout en ayant une vitesse de libération de l'huile améliorée.

La demanderesse a trouvé de manière surprenante que l'addition de certaines particules solides minérales ou organiques permettait d'accélérer la vitesse de libération d'huile et d'améliorer l'efficacité des compositions.

La présente invention a donc pour objet une composition pour application topique sous forme d'émulsion huile-dans-eau, caractérisée par le fait qu'elle comporte :

- Une phase lipophile (A), présente en une quantité d'au moins 60 % en poids par rapport au poids total de la composition,
- une phase aqueuse (C) présente en une quantité inférieure ou égale à 30 % en poids par rapport au poids total de la composition,
- un système émulsionnant (B) présent en une quantité de 2 à 20 % en poids par rapport au poids total de la composition et comprenant au moins un émulsionnant ayant un HLB allant de 8 à 18,
- de 0,5 à 10 % en poids par rapport au poids total de la composition, d'une ou plusieurs charges ayant une prise d'huile égale ou supérieure à 75 ml/100 g,
- le rapport système émulsionnant (B) / la phase lipophile (A) allant de 0,04 à 0,2.
- le diamètre moyen en volume des gouttelettes de phase huileuse D[4,3] va de 0,09 $\mu$m à 4 $\mu$m.

Du fait qu'elle est destinée à une application topique, la composition de l'invention contient un milieu physiologiquement acceptable. Par "milieu physiologiquement acceptable", on entend un milieu convenant à une application topique sur la peau ou les phanères, c'est-à-dire compatible avec la peau, les muqueuses, les lèvres, les cils, les yeux, les cheveux et les ongles. Cette composition peut constituer notamment une composition cosmétique ou dermatologique.

On entend par « phase lipophile » dans la présente demande, la phase contenant les composés lipophiles que sont notamment les huiles (constituants lipophiles liquides à température ambiante), les gommes, les pâtes et les cires. Ce sont par exemple des triglycérides, des hydrocarbures, des esters, des éthers, des silicones, comme décrit plus loin, et tous les additifs lipophiles éventuellement présents. Les émulsionnants et co-émulsionnants du système émulsionnant ne font pas partie de la phase lipophile telle que définie ci-dessus.

Il est important d'avoir une quantité suffisante de phase lipophile et notamment d'huiles pour obtenir une texture crémeuse, et le problème à la base de l'invention a été la difficulté d'obtenir une composition de texture crémeuse avec des petites tailles de globules, qui contienne suffisamment d'huiles tout en étant néanmoins bien stable.

On entend par « charges » dans la présente demande, des particules solides qui peuvent être minérales ou organiques. La prise d'huile, caractéristique de ces particules solides utilisées dans la composition selon l'invention est déterminée selon la norme AFNOR NF T30-022 (mai 1972), comme détaillé ci-après.

Les émulsions H/E selon l'invention sont obtenues par la technologie d'inversion de phase en température et se caractérisent par :

- leur viscosité : il s'agit principalement de crèmes,
- leur aspect qui peut aller de l'opaque au translucide,
- le pH qui va de 3 à 8,
- la petite taille des gouttelettes de phase huileuse,
- leur grande vitesse de libération d'huile (ou vitesse de claquage) comme ce sera démontré ci-après,
- lorsqu'elles sont rincées, une bonne ré-émulsification de l'huile lors du rinçage à l'eau pour limiter le résidu lipophiles sur la peau ou les cheveux.

En outre, ces émulsions sont agréables à utiliser du fait de la phase aqueuse externe et elles allient ainsi efficacité et agrément cosmétique.

Les compositions peuvent être utilisées de manière différente en fonction des applications souhaitées : elles peuvent être par exemple essuyées ou rincées à l'eau pour un produit solaire, ou bien essuyées ou rincées avec un tonique pour un produit démaquillant, ou bien encore rincées à l'eau pour un produit de soin capillaire après shampooing.

Les compositions selon l'invention peuvent se présenter sous forme de crèmes plus ou moins épaisses, opaques à translucides, et elles peuvent s'écouler ou non sous leur propre poids selon leur viscosité. Pour une crème, la viscosité mesurée à 25°C avec l'appareil de mesure Rheomat 180 à 200 rpm (tours par minute) doit être égale ou supérieure à 1 Pa.s. Le Rheomat 180 est équipé d'un mobile différent selon les viscosités, par exemple d'un mobile 3 pour la gamme des viscosités de 0,2 à 4 Pa.s, et d'un mobile 4 pour la gamme des viscosités supérieures à 2 Pa.s. Mesurée dans les conditions indiquées ci-dessus, la viscosité des compositions de l'invention peut aller par exemple de 1 à 30 Pa.s et de préférence de 1 à 20 Pa.s. Cette viscosité est mesurée généralement 10 minutes après la mise rotation du mobile.

Quand la viscosité est mesurée par un appareil Haake RS 150, déterminée par des mesures en écoulement à 0,1 s$^{-1}$, elle peut aller de 500 à 5000 Pa.

La taille moyenne des gouttelettes de phase huileuse sont mesurées par diffraction de la lumière à l'aide d'un granulomètre Mastersizer 2000 (commercialisés par Malvern Instruments). Ces mesures sont effectuées sur l'émulsion diluée dans une solution de SDS (Sodium Dodecylsulfate) à 1 % dans l'eau. Un logiciel permet d'accéder au diamètre moyen en volume D[4,3] ($\mu$m) (Voir Operators Guide, Malvern Instruments, Decembre 1998, p.61 à 67).

La taille moyenne D[4,3] ($\mu$m) des gouttelettes de phase huileuse de la composition de l'invention varie de 0,09 $\mu$m à 4 $\mu$m, plus particulièrement de 0,1 $\mu$m à 2 $\mu$m et de préférence de 0,1 $\mu$m à 1 $\mu$m.

Système émulsionnant

Le système émulsionnant (B) utilisé dans la composition selon l'invention comprend un ou plusieurs émulsionnants dont la solubilité dans l'huile augmente avec l'augmentation de la température, émulsionnants permettant d'obtenir des émulsions par inversion de phase en température. La HLB (hydrophilic lipophilic balance) de ces émulsionnants va de 8 à 18 et de préférence de 10 à 16. Ils peuvent être choisis notamment parmi les alcools gras éthoxylés, les acides gras éthoxylés, les glycérides partiels d'acides gras éthoxylés, les triglycérides d'acides gras polyglycérolés et leurs dérivés éthoxylés, et leurs mélanges.

Les émulsionnants sont de préférence choisis parmi les alcools gras éthoxylés ou les acides gras éthoxylés ayant les formules (I) et (II) suivantes :

$$R\text{-}O\text{-}(CH_2\text{-}CH_2\text{-}O)_mH \qquad (I)$$

$$R\text{-}COO\text{-}(CH_2\text{-}CH_2\text{-}O)_mH \qquad (II)$$

où R est une chaîne hydrocarbonée linéaire ou ramifiée, saturée ou insaturée, ayant de 10 à 24 atomes de carbone, et m est un nombre entier allant de 8 à 50.

Comme alcools gras éthoxylés, on peut citer par exemple les produits d'addition d'oxyde d'éthylène avec l'alcool laurylique, notamment ceux comportant de 9 à 50 groupes oxyéthylénés (Laureth-9 à Laureth-50 en noms CTFA) ; les produits d'addition d'oxyde d'éthylène avec l'alcool béhénylique, notamment ceux comportant de 9 à 50 groupes oxyéthylénés (Beheneth-9 à Beheneth-50 en noms CTFA) ; les produits d'addition d'oxyde d'éthylène avec l'alcool cétéarylique (mélange d'alcool cétylique et d'alcool stéarylique), notamment ceux comportant de 9 à 30 groupes oxyéthylénés (Ceteareth-9 à Ceteareth-30 en noms CTFA) ; les produits d'addition d'oxyde d'éthylène avec l'alcool cétylique, notamment ceux comportant de 9 à 30 groupes oxyéthylénés (Ceteth-9 à Ceteth-30 en noms CTFA) ; les produits d'addition d'oxyde d'éthylène avec l'alcool stéarylique, notamment ceux comportant de 9 à 30 groupes oxyéthylénés (Steareth-9 à Steareth-30 en noms CTFA) ; les produits d'addition d'oxyde d'éthylène avec l'alcool isostéarylique, notamment ceux comportant de 9 à 50 groupes oxyéthylénés (Isosteareth-9 à Isosteareth-50 en noms CTFA) ; et leurs mélanges.

Comme acides gras éthoxylés, on peut citer par exemple les produits d'addition d'oxyde d'éthylène avec les acides laurique, palmitique, stéarique ou béhénique, et leurs mélanges, notamment ceux comportant de 9 à 50 groupes oxyéthylénés tels que les laurates de PEG-9 à PEG-50 (en noms CTFA : PEG-9 laurate à PEG-50 laurate) ; les palmitates de PEG-9 à PEG-50 (en noms CTFA : PEG-9 palmitate à PEG-50 palmitate) ; les stéarates de PEG-9 à PEG-50 (en noms CTFA : PEG-9 stéarate à PEG-50 stearate) ; les palmito-stéarates de PEG-9 à PEG-50 ; les béhénates de PEG-9 à PEG-50 (en noms CTFA : PEG-9 behenate à PEG-50 behenate) ; et leurs mélanges.

On peut utiliser aussi des mélanges de ces dérivés oxyéthylénés d'alcools gras et d'acides gras.

De préférence, le système émulsionnant de la composition de l'invention contient comme émulsionnant au moins un alcool gras éthoxylé, et plus particulièrement le beheneth-10.

Le système émulsionnant peut contenir en outre un ou plusieurs co-émulsionnants. Comme co-émulsionnants, on peut citer par exemple les alcools gras ayant 8 à 30 atomes de carbone, comme par exemple l'alcool cétylique, l'alcool stéarylique, l'alcool béhénique ; les acides gras ayant 8 à 30 atomes de carbone, comme par exemple l'acide palmitique, l'acide stéarique, l'acide béhénique ; les esters gras de glycérol, comme par exemple le glycéryl stéarate ; les dérivés oxyéthylénés de ces alcools gras, acides gras et esters gras de glycérol, comportant 2 à 8 groupes oxyde d'éthylène, et leurs mélanges.

Le système émulsionnant est présent en une quantité allant de 2 à 20 %, de préférence de 3 à 16 % et mieux de 3 à 11 % en poids par rapport au poids total de la composition.

Le rapport système émulsionnant (B) / phase lipophile va de 0,04 à 0,2, de préférence de 0,06 à 0,18. Comme indiqué plus haut, on entend par « phase lipophile » l'ensemble des constituants qui ne sont pas hydrophiles et qui sont différents des émulsionnants ou co-émulsionnants du système émulsionnant.

Phase lipophile

La phase lipophile appelée aussi phase huileuse ou grasse, est constituée des constituants lipophiles, c'est-à-dire huiles et autres corps lipophiles présents dans la composition, ainsi que de tous additifs lipophiles éventuellement présents. La phase lipophile contient au moins une huile, notamment une huile cosmétique.

On entend par "huile" un corps gras liquide à la température ambiante (25°C).

La phase lipophile est présente en une quantité d'au moins 60 % en poids par rapport au poids total de la composition. La quantité de phase lipophile peut aller par exemple de 60 à 80 % en poids, de préférence de 61 à 75 % en poids et mieux de 62 à 70 % en poids par rapport au poids total de la composition. La phase lipophile peut ne comprendre que des huiles (corps gras liquides) ou elle peut comprendre un mélange d'huiles et d'autres corps gras. Toutefois, de préférence, la quantité d'huiles est d'au moins 50 % en poids par rapport au poids total de la composition, et de préférence d'au moins 60 % par rapport au poids total de la composition.

Comme huiles plus particulièrement utilisables dans la composition de l'invention, on peut citer par exemple :

- les huiles hydrocarbonées d'origine animale, telles que le perhydrosqualène (ou squalane) ;
- les esters et les éthers de synthèse, notamment d'acides gras, comme les huiles de formules $R^1COOR^2$ et $R^1OR^2$ dans laquelle $R^1$ représente le reste d'un acide gras comportant de 8 à 29 atomes de carbone, et $R^2$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le düsostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; les esters du pentaérythritol comme le tétraisostéarate de pentaérythrytyle ; les dérivés lipophiles d'acides aminés, tels que le lauroyl sarcosinate d'isopropyle (nom INCI : Isopropyl Lauroyl sarcosinate) commercialisé sous la dénomination Eldew SL 205 par la société Ajinomoto ;
- les hydrocarbures linéaires ou ramifiés, d'origine minérale ou synthétique, tels que les huiles minérales (mélange d'huiles hydrocarbonées dérivées du pétrole ; nom INCI : Mineral oil), les huiles de paraffine, volatiles ou non, et leurs dérivés, la vaseline, les polydécènes, l'isohexadecane, l'isododecane, l'isoparaffine hydrogéné tel que l'huile de Parléam® commercialisée par la société NOF Corporation (nom INCI ; Hydrogenated Polyisobutene) ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclopentasiloxane et la cyclohexadiméthylsiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- les huiles fluorées telles que celles partiellement hydrocarbonées et/ou siliconées comme celles décrites dans le document JP-A-2-295912 ;
- les éthers tels que l'éther dicaprylique (nom CTFA : Dicaprylyl ether) ; et les benzoates d'alcools gras en $C_{12}$-$C_{15}$ (Finsolv TN de FINETEX) ;
- leurs mélanges.

Les autres constituants lipophiles pouvant être présents dans la phase huileuse sont par exemple les huiles végétales, les cires ; les gommes telles que les gommes de silicone (diméthiconol) ; les résines de silicone telles que la trifluorométhyl-C1-4-alkyldimethicone et la Trifluoropropyldimethicone ; et leurs mélanges.

Quand la composition de l'invention est utilisée comme composition démaquillante, elle contient de préférence au moins une huile démaquillante, à laquelle on peut ajouter une ou plusieurs autres huiles démaquillantes ou non.

Les huiles démaquillantes peuvent être choisies notamment parmi les hydrocarbures ramifiés, d'origine minérale ou synthétique, décrits ci-dessus, les esters gras et leurs mélanges.

Les esters gras sont de préférence ceux obtenus à partir d'un alcool à chaîne linéaire ou ramifiée, ayant de 1 à 17 atomes de carbone et d'un acide gras à chaîne linéaire ou ramifiée, ayant de 3 à 18 et de préférence de 12 à 17 atomes de carbone.

Comme esters gras utilisables comme huiles démaquillantes, on peut citer plus particulièrement le myristate de butyle, le laurate de butyle, le stéarate de butyle, le stéarate d'isopropyle, l'isostéarate d'isotéaryle, le palmitate d'ethyl-2 hexyle (ou palmitate d'octyle), le pelargonate d'ethyl-2 hexyle (ou pelargonate d'octyle), le stéarate d'éthyl-2 hexyle (ou stéarate d'octyle), le myristate d'octyl-2 dodécyle, l'hydroxystéarate d'éthyl-2 hexyle (ou hydroxystéarate d'octyle), le laurate d'isopropyle, le myristate d'isopropyle, le palmitate d'isopropyle, le palmitate d'isobutyle, le stéarate d'isobutyle, le stéarate d'isocétyle, l'adipate de diisopropyle, l'adipate de di-éthyl-2 hexyle (ou adipate de di-octyle), l'adipate de diisocetyle, le succinate d'ethyl-2 hexyle (ou succinate d'octyle), le sébacate de diisopropyle, le malate d'ethyl-2 hexyle (ou malate d'octyle), le caprate/caprylate de pentaérythritol, le pentaérythritol de tétraisostéarate, l'hexanoate d'éthyl-2 hexyle (ou hexanoate d'octyle), le laurate d'éthyle, le myristate de méthyle, l'octanoate d'octyldodécyle, le néopentanoate d'isodécyle, le néopentanoate d'isostéaryle, l'isononanoate de cétéaryle, l'isononanoate d'isodécyle, l'isononanoate d'isononyle, l'isononanoate d'isotridécyle, le lactate de lauryle, le lactate de myristyle, le lactate de cétyle, le myristate d'éthyle, le propionate de myristyle, l'éthyl-2 hexanoate d'éthyl-2 hexyle (ou éthyl-2 hexanoate d'octyle), l'octanoate d'éthyl-2 hexyle (ou octanoate d'octyle), le caprate/caprylate d'éthyl-2 hexyle (ou caprate/caprylate d'octyle), le monococoate d'éthyl-2 hexyle (ou monococoate d'octyle), le palmitate de méthyle, le myristate d'isobutyle, le palmitate d'éthyle, le laurate d'isohexyle, le laurate d'hexyle, l'isostéarate d'isopropyle, et leurs mélanges.

Selon un mode particulier de réalisation de l'invention, quand la composition selon l'invention constitue une composition démaquillante, elle contient un ou plusieurs esters gras choisis parmi ceux cités ci-dessus.

Comme hydrocarbures ramifiés, d'origine minérale ou synthétique, pouvant être utilisés dans la composition selon l'invention constituant une composition démaquillante, on peut citer notamment l'isoparaffine, l'isohexadecane, l'isododecane, le polyisobutène hydrogéné tel que l'huile de Parléam®.

Phase aqueuse

La composition selon l'invention comprend une quantité de phase aqueuse inférieure ou égale à 30 % du poids total de la composition, cette quantité pouvant aller par exemple de 10 à 30 % en poids et de préférence de 15 à 30 % en poids par rapport au poids total de la composition. La quantité d'eau dans la phase aqueuse peut aller par exemple de 5 à 30 % en poids et de préférence de 10 à 25 % en poids par rapport au poids total de la composition.

De manière classique, la phase aqueuse peut contenir, outre l'eau, un ou plusieurs solvants hydrosolubles choisis parmi les polyols (ou alcools polyhydriques), les alcool(s) inférieur(s) hydrosoluble(s) et leurs mélanges. On entend par alcool inférieur, un alcool comportant de 1 à 8 atomes de carbone. Comme polyols, on peut citer par exemple la glycérine ; les glycols comme le propylène glycol, le butylène glycol, l'isoprène glycol et les polyéthylène glycols tels que le PEG-8 ; le sorbitol ; les sucres tels que le glucose, le fructose, le maltose, le lactose, le sucrose ; et leurs mélanges. Comme alcools inférieurs, on peut citer par exemple l'éthanol, l'isopropanol, le butanol et leurs mélanges. Lorsqu'ils sont présents dans la composition de l'invention, le ou les solvant(s) peuvent être en une quantité allant de 0,01 à 60 % en poids, de préférence de 0,5 à 50 % en poids et mieux de 5 à 20 % en poids par rapport au poids total de la phase aqueuse. Ces adjuvants ainsi que leurs concentrations doivent être tels qu'ils ne modifient pas la propriété recherchée pour la composition de l'invention.

Charges minérales ou organiques

La composition selon l'invention contient une ou plusieurs charges (particules solides) minérales ou organiques ayant une prise d'huile égale ou supérieure à 75 ml/100 g.

La demanderesse a constaté de manière surprenante que seule l'addition de telles charges permettait d'avoir une meilleure vitesse de libération de l'huile lors du massage sur le support d'application (peau, zone autour de l'oeil, muqueuses, cuir chevelu, cils, cheveux).

Les charges utilisées dans la composition selon l'invention sont caractérisées par leur prise d'huile, c'est-à-dire par leur capacité à adsorber de l'huile de ricin. La prise d'huile est déterminée par une méthode analogue à la norme AFNOR NF T30-022, l'huile de lin de cette norme étant remplacée ici par l'huile de ricin. La prise d'huile correspond à la quantité d'huile de ricin (en ml) nécessaire pour obtenir une pâte ferme et homogène avec 100 g de charges. Plus cette valeur est grande, plus la charge adsorbe l'huile.

Pour déterminer la prise d'huile selon la méthode utilisée, analogue à la norme AFNOR NF T30-022, on prend une quantité m (poids) de charge (1 à 20 grammes) appropriée selon la prise d'huile probable de la charge, comme décrit dans la norme AFNOR. On place cette quantité de charge sur une plaque et on ajoute lentement de l'huile de ricin, à raison de quatre à cinq gouttes à la fois, au moyen d'une pipette. Après chaque addition, on incorpore l'huile dans la charge avec un couteau à palette, et on continue d'ajouter l'huile à cette cadence jusqu'à la formation de conglomérats d'huile et de charge. A partir de ce moment, on ajoute l'huile goutte à goutte et on fait suivre chaque addition d'une trituration énergique avec le couteau. On cesse l'addition quand on obtient une pâte ferme et lisse, qui se laisse étendre sans craquelures ni formation de grumeaux. On note alors la quantité m2 d'huile utilisée en gramme.

La prise d'huile en ml/100 g correspond à :

$$\frac{m_2 \times 100}{\rho_2 \times m}$$

$\rho_2$ étant la masse volumique de l'huile de ricin = 0,96 (référence. de la masse volumique de l'huile de ricin : Manuel des corps gras de JP Wolff, Editeur Azoulay Paris 1968, page 360).

On fait deux mesures et on prend la moyenne.

Les charges améliorant la vitesse de libération de l'huile possèdent une prise d'huile supérieure à 75 ml/100 g et de préférence supérieure ou égale à 100 ml/100g.

L'addition de charges minérales ou organiques ayant une prise d'huile égale ou supérieure à 75 ml/100 g, permet d'améliorer la quantité d'huile libérée, et notamment d'améliorer le pouvoir démaquillant de la composition quand elle est utilisée pour le démaquillage de la peau ou des cils.

Toutes les charges minérales ou organiques ne permettent pas de libérer l'huile rapidement. Ainsi, des charges telles que le talc LUZENAC 15 M00® (silicates de magnésium) commercialisé par la société LUZENAC ou de l'amidon de maïs, AMIDON DE MAIS B® commercialisé par Roquette Frères ne modifient pas la vitesse de libération des huiles ou peuvent même la ralentir, comme c'est le cas par exemple avec 4% de talc, comme le montrent les exemples comparatifs présentés plus loin. Ainsi, seules les charges minérales ou organiques choisies selon les critères décrits ci-dessus permettent d'améliorer la vitesse de libération des huiles et d'obtenir une meilleure efficacité des émulsions fines H/E contenant une grande quantité d'huiles.

La ou les charges utilisées dans la composition selon l'invention peuvent être minérales et organiques, poreuses ou non, sphériques ou non.

a- Charges minérales

Comme charges minérales pouvant être utilisées dans la composition de l'invention, on peut citer par exemple les oxydes de silicium telles que les silices ayant une surface spécifique égale ou supérieure à 120 $m^2$/g. On entend par «silice» aussi bien les silices pures, hydrophiles ou hydrophobes, que les particules enrobées de silice. Ces silices sont de préférence amorphes et elles peuvent être d'origine pyrogénée ou d'origine précipitée. Elles peuvent se présenter sous forme pulvérulente ou en dispersion aqueuse. Elles sont généralement caractérisées par une surface spécifique allant de 120 à 800 $m^2$/g, une dimension moyenne de particules élémentaires en nombre allant de 3 à 50 nm, une densité tassée allant de 40 à 200 g/l et mieux de 50 à 150 g/l, et une taille d'agrégats allant de 10 à 300 $\mu$m.

Comme silices de ce type, on peut citer par exemple les silices commercialisées sous les dénominations AEROSIL 150®, 200®, 300®, 380®, FK 320 DS®, R805®, R812®, R974® par la Société Degussa-Hüls, les silices commercialisées sous les dénominations Sunsphères H-31®, H-32®, H-33®, H-51®, H52®, H-121® ou H-122®, ayant une prise d'huile allant de 150 à 400 ml/100g et une surface spécifique variant de 700 à 800 $m^2$/g, par la Société Dohkai Chemical Industries.

b- Charges organiques

Comme charges organiques pouvant être utilisées dans la composition de l'invention, on peut citer par exemple les microsphères de polyamide (Nylon) ayant une taille moyenne en nombre allant de 5 $\mu$m à 30 $\mu$m, comme par exemple celles commercialisées sous les dénominations ORGASOL 2002 D NAT COS®, ORGASOL 2002 UD NAT COS®, ORGASOL 2002 EXD NAT COS® par la société ATOFINA, celles commercialisées sous les dénominations MICROPAN 777®, MICROFINE-COPOLYAMIDE 6/12® par la société CENTERCHEM, celles commercialisées sous la dénomination GANZPEARL GPA-700® par la société SPCI.

On peut utiliser des mélanges de ces charges organiques et minérales.

La ou les charges sont de préférence ajoutées à la composition après préparation de l'émulsion.

La quantité de charges peut aller de 0,5 à 10 % en poids, de préférence de 0,5 à 5 % en poids par rapport au poids total de la composition. Le rapport charges / constituants lipophiles va de préférence de 0,006 à 0,08.

Additifs

La composition selon l'invention peut aussi contenir tout adjuvant ou additif habituellement utilisé dans les domaines considérés et notamment dans les domaines cosmétique ou dermatologique.

Parmi les adjuvants classiques susceptibles d'être contenus dans la phase aqueuse et/ou dans la phase lipophile des émulsions conformes à l'invention (selon le caractère hydrosoluble ou liposoluble de ces adjuvants), on peut citer notamment les tensioactifs moussants anioniques (tels que lauryl ether sulfate de sodium, alkyl phosphate de sodium, trideceth sulfate de sodium), amphotères (tels que alkyl bétaïne, disodium cocoamphodiacetate) ou non ioniques de HLB supérieure à 10 (tels que POE/PPG/POE, Alkylpolyglucoside, polyglyceryl-3 hydroxylauryl ether) ; les conservateurs ; les séquestrants (EDTA) ; les antioxydants ; les parfums ; les matières colorantes telles que les colorants solubles, les pigments et les nacres ; les charges matifiantes, tenseurs, blanchissantes ou exfoliantes ; les filtres solaires ; les actifs cosmétiques ou dermatologiques et agents ayant pour effet d'améliorer les propriétés cosmétiques de la peau, hydrophiles ou lipophiles ; les électrolytes. Les quantités de ces différents adjuvants sont celles classiquement utilisées dans le domaine considéré, et par exemple de 0,01 à 20 % du poids total de la composition.

Comme actifs utilisables dans la composition de l'invention, on peut citer par exemple, les vitamines hydrosolubles ou liposolubles comme la vitamine A (rétinol), la vitamine E (tocophérol), la vitamine C (acide ascorbique), la vitamine B5 (panthénol), la vitamine B3 (niacinamide), les dérivés de ces vitamines (notamment esters) et leurs mélanges ; les antiseptiques ; les actifs anti-bactériens comme le 2,4,4'-trichloro-2'-hydroxy diphényl éther (ou triclosan), le 3,4,4'-trichlorocarbanilide (ou triclocarban) ; les antisébohrréïques ; les antimicrobiens tels que le peroxyde de benzoyle, l'acide salicylique, le triclosan, l'acide azélaïque, la niacine (vit. PP) ; les amincissants tels que la caféine ; les azurants optiques, et tout actif approprié pour le but final de la composition, et leurs mélanges.

La quantité d'actifs dépend du but recherché. Le ou les actifs peuvent être par exemple présents en une concentration allant de 0,001 à 20 %, de préférence de 0,01 à 10 % en poids et mieux de 0,05 à 5 % du poids total de la composition.

Bien entendu, l'homme de l'art veillera à choisir le ou les éventuels additifs de la composition selon l'invention de manière telle que les propriétés avantageuses attachées intrinsèquement à la composition conforme à l'invention ne soient pas ou substantiellement pas, altérées par l'addition envisagée.

Les émulsions selon l'invention sont obtenues généralement par un procédé d'inversion de phase. Ce procédé de préparation consiste à :

- 1) Peser dans un récipient tous les constituants de la composition (à l'exception des matières premières thermosensibles et des charges)
- 2) Homogénéiser le mélange, par exemple au moyen d'un Rayneri 350 tours/min, et chauffer en augmentant progressivement la température au moyen d'un bain marie jusqu'à une température supérieure ou égale à la température d'inversion de phase T2, c'est-à-dire jusqu'à l'obtention d'une phase transparente ou translucide (zone de microémulsion ou de phase lamellaire) puis d'une phase blanche plus visqueuse qui indique l'obtention de l'émulsion inverse (E/H).
- 3) Arrêter le chauffage et maintenir l'agitation jusqu'à retour à la température ambiante, en passant par la température d'inversion de phase T1, c'est-à-dire la température à laquelle se forme une émulsion H/E fine.
- 4) Lorsque la température est redescendue en dessous de la zone d'inversion de Phase en Température (T1), additionner les charges et éventuellement les matières premières thermosensibles.

On obtient une émulsion H/E stable dont les gouttelettes d'huile sont fines.

Dans la zone de formation d'une microémulsion (mélange translucide), les interactions hydrophiles et hydrophobes sont équilibrées car la tendance du tensioactif est de former aussi bien des micelles directes que des micelles inverses. Par chauffage au-delà de cette zone, il y a formation d'une émulsion E/H (mélange opaque blanc), car le tensioactif favorise la formation d'une émulsion eau dans huile. Puis lors du refroidissement en dessous de la zone d'inversion de phase, l'émulsion devient une émulsion H/E.

L'émulsification par inversion de phase est expliquée en détail dans l'ouvrage T.Förster, W von Rybinski, A.Wadle, Influence of microemulsion phases on the préparation of fine disperse emulsions, Advances in Colloid and interface sciences, 58, 119-149, 1995 cité ici pour référence.

Les compositions selon l'invention se présentent sous forme de crèmes plus ou moins souples, et elles peuvent constituer notamment des compositions cosmétiques ou dermatologiques, par exemple des compositions cosmétiques pour le traitement des matières kératiniques telles que la peau, les muqueuses, les cils, les cheveux et les ongles. Elles peuvent constituer aussi par exemple des compositions de démaquillage et/ou de nettoyage et/ou de soin de la peau, des muqueuses telles que les lèvres, et/ou des cils, des compositions pour le massage de la peau du corps ou du visage, des compositions gommantes (ou exfoliantes) aussi bien pour le visage que pour les mains (lorsque la composition contient des particules exfoliantes), des compositions solaires (protection U.V.), et après-solaires. Elles peuvent constituer encore des compositions de maquillage des matières kératiniques et notamment la peau, les lèvres et les cils, et plus particulièrement comme de fonds de teint, rouges à lèvres, brillants à lèvres (gloss) après addition de pigments et/ou charges appropriés.

Les compositions selon l'invention peuvent être aussi utilisées comme baumes de soin de douche (à rincer, par exemple en massant le produit jusqu'à libération de l'huile, puis en rinçant la peau qui est alors douce et hydratée) ; comme après-shampooings et baumes de soin capillaire ; comme produits de rasage ; comme masques y compris comme masque réparateur après-soleil ; comme cataplasme amincissant sur zone "capitons" (à masser puis à rincer) ; comme baume de massage ; comme baume réparateur lèvres à rincer ; comme baume pour pieds secs. Lors de ces utilisations, le produit est ensuite rincé.

Lorsque la composition est un produit exfoliant (appelé aussi produit descincrustant), l'utilisation consiste à appliquer le produit sur le visage ou les mains ou le corps, à frotter une minute ou deux puis à rincer. La peau est alors lisse, douce et désincrustée.

Ainsi, la présente invention a pour objet l'utilisation cosmétique de la composition telle que définie ci-dessus, pour le traitement cosmétique des matières kératiniques.

Ainsi, la présente invention a aussi pour objet l'utilisation cosmétique de la composition telle que définie ci-dessus, pour le démaquillage et/ou le nettoyage de la peau, des lèvres et/ou des cils.

La présente invention a aussi pour objet l'utilisation cosmétique de la composition telle que définie ci-dessus, pour le maquillage de la peau, des lèvres et/ou des cils.

La présente invention a encore pour objet un procédé cosmétique de démaquillage et/ou de nettoyage de la peau, y compris du cuir chevelu, des cils et/ou des lèvres, caractérisé par le fait que l'on applique sur la peau, les cils et/ou les lèvres, une composition telle que définie ci-dessus.

Les exemples indiqués ci-dessous permettront de mieux comprendre l'invention sans toutefois présenter un caractère limitatif. Les quantités indiquées sont en % en poids sauf mention contraire. Les noms sont en noms chimiques et en noms CTFA selon les composés. Dans certains exemples, les températures T1 et T2 correspondant aux températures bordant la zone d'inversion de phase sont indiquées.

Les exemples réalisés ont été soumis à des tests permettant de mesurer leurs avantages pour le démaquillage. Ces tests qui portent sur le pourcentage de démaquillage et la vitesse de libération de l'huile (ou vitesse de claquage) ont été réalisés selon les protocoles suivants :

I. Protocole de démaquillage in Vivo

I.a. Matériel

1. Huile démaquillante rinçable à l'eau : « Huile Démaquillante fraîche » de Shu Uemura
2. Savon de Marseille
3. 1 cache comportant une partie centrale vide découpée dans une fiche bristol de 4x4 cm + 1 feutre LUMICOLOR permanent S
4. Colorimètre CR300 (mesure de la colorimétrie en L.a.b)
5. Fond de teint sans transfert Air Wear « Sable » LSF 12 de L'Oreal
6. 8 petits verres de montre + des petites spatules
7. 1 balance de précision
8. 1 chronomètre

I.b. Protocole

- On trace sur la peau 4 zones (2 par bras) de la dimension de la partie centrale découpée du cache, à l'aide du feutre et du cache ;
- On nettoie la peau de ces zones à l'huile démaquillante rinçable puis au savon, on rince et on sèche (les marques de feutre sont alors atténuées pour éviter tout transfert de feutre dans le fond de teint au moment de l'application) ;
- On mesure par colorimétrie la peau nue : 3 mesures de L,a,b par zone (tête de lecture du colorimètre positionnée parfaitement au centre de la zone),
- On applique dans les 4 zones délimitées, de façon homogène, 16 mg+/- 0,05 mg de fond de teint et on attend 30 minutes ;
- On mesure par colorimétrie la peau avec fond de teint : 3 mesures de L,a,b par zone ;
- On pèse dans les verres de montre 4 fois, 208 mg+/-1 mg de composition démaquillante à tester ;
- On démaquille chaque zone au doigt par mouvement circulaire pendant 10 secondes puis on rince à l'eau du robinet (tiède ; dureté non contrôlée) en effleurant légèrement avec les doigts de la main disponible. On fait les 4 zones sans attendre.
- On tamponne avec un mouchoir jetable type kleenex et on attend 15 minutes (sensation de peau sèche) ;
- On mesure par colorimétrie la peau démaquillée : 3 mesures de L,a,b par zone.

Le calcul du pourcentage de démaquillage est fait de la manière suivante :

- On détermine l'écart colorimétrique de la peau maquillée (peau FdT) par rapport à la peau nue, $\Delta E_{max}$ étant la valeur correspondant à un démaquillage parfait :

$$\Delta E \max = \sqrt{(\Delta a_1^2 + \Delta b_1^2 + \Delta L_1^2)}$$

avec

$\Delta a_1$ = a peau nue - a peau FdT
$\Delta b_1$ = b peau nue - b peau FdT
$\Delta L_1$ = L peau nue - L peau FdT

- On détermine pour la composition démaquillante testée, l'écart colorimétrique $\Delta E$ de la peau démaquillée (peau démaq) par rapport à la peau maquillée (peau FdT) pour chaque zone:

$$\Delta E = \sqrt{(\Delta a_2^2 + \Delta b_2^2 + \Delta L_2^2)}$$

avec

$\Delta a_2$ = a peau démaq - a peau FdT
$\Delta b_2$ = b peau démaq - b peau FdT
$\Delta L_2$ = L peau démaq - L peau FdT

- On détermine le rapport $\Delta E / \Delta E \max$.
- on calcule le pourcentage de démaquillage moyen qui est la moyenne des 4 valeurs de $(\Delta E / \Delta E \max) \times 100$ pour la composition démaquillante testée.

II. Mesure de la vitesse de libération de l'huile (vitesse de claquage)

II.a. Matériel

1. Huile démaquillante rinçable à l'eau : « Huile Démaquillante fraîche » de Shu Uemura
2. Savon de Marseille
3. 1 cache avec une partie centrale carrée découpée dans une fiche bristol de 4x4 cm + 1 feutre LUMICOLOR permanent S
4. 4 petits verres de montre + des petites spatules
5. 1 balance de précision
6. 1 chronomètre

II.b. Protocole

- On trace sur la peau 4 zones (2 par bras) de la dimension de la partie centrale découpée du cache, à l'aide du feutre et du cache ;
- On nettoie la peau de ces zones au savon, on rince et on sèche (les marques de feutre sont alors atténuées pour éviter tout transfert de feutre dans le fond de teint au moment de l'application) ;
- On applique dans une des zones délimitées, de façon homogène, une quantité M de produit démaquillant, M en mg étant 100 mg+/- 1 mg de produit démaquillant ;
- on déclenche le chronomètre
- on masse de façon homogène, et on note le temps écoulé pour que l'huile soit libérée, c'est-à-dire au moment où le produit devient subitement plus glissant sous les doigts.

Si t est le temps, en secondes, écoulé pour que l'huile soit libérée, la vitesse de claquage (libération de l'huile) est égale à : $t \times 100 / M$.
On renouvelle le test sur les trois autres zones et on fait la moyenne des résultats.
A côté des exemples selon l'invention, on a réalisé des exemples comparatifs qui ne remplissent pas les conditions des compositions selon l'invention, et on a mesuré leur viscosité à 25°C, leur pH, le pourcentage de démaquillage et la vitesse de claquage.

**I. Exemples avec silice (exemple 1 selon l'invention) et sans silice (exemple comparatif 1)**

| Composition | Exemple 1 selon l'invention | Exemple comparatif 1 (sans charge) |
|---|---|---|
| EUMULGIN BA 10® (1) | 5 | 5 |
| Isopropyl palmitate® (2) | 34,5 | 35 |
| Monococoate d'ethyl 2 hexyle (3) | 34,5 | 35 |
| Propyl paraben | 0,15 | 0,15 |
| Methyl paraben | 0,15 | 0,15 |
| glycérine | 10 | 10 |
| Eau permutée | 14,45 | 14,45 |
| Chlorhexidine digluconate | 0,25 | 0,25 |
| AEROSIL 200® (4) | 1 | - |
| total | 100 | 100 |
| Total TA | 5 | 5 |
| Total huile | 70 | 70 |
| Rapport TA/Huile | 0,07 | 0,07 |
| T1 (°C) | 69 | 73 |
| T2 (°C) | 77 | 85 |
| Aspect | Crème souple, blanche, légèrement translucide | Crème souple, blanche, légèrement translucide |
| Viscosité (Pa.s) | 6,6 | 5 |
| pH | 5,9 | 5,8 |
| Vitesse de libération de l'huile (s/100mg) | 5 | 23 |
| % démaquillage | 96 | 90 |

(1) EUMULGIN BA 10® commercialisé par Cognis = Alcool béhénylique oxyéthyléné (10 OE)
(2) ISOPROPYL PALMITATE® commercialisé par Cognis = palmitate d'isopropyle
(3) Monococoate d'ethyl-2 hexyle® commercialisé par Stéarineries Dubois
(4) AEROSIL 200® commercialisé par Degussa-Hüls = Silice pyrogénée hydrophile (prise d'huile : 460 ml/100g ; Densité tassée : 50g/l ; 200 m$^2$/g)

Il ressort de ce tableau que l'addition de la silice utilisée selon l'invention améliore nettement la vitesse de libération de l'huile (appelée aussi « vitesse de claquage ») lors de l'application sur la peau et l'efficacité de démaquillage de la composition de l'invention.

**II. Exemples avec particules de Nylon (exemple 2 selon l'invention) et sans particules de Nylon (exemple comparatif 2)**

| Composition | Exemple 2 selon l'invention | Exemple comparatif 2 (sans charge) |
|---|---|---|
| EUMULGIN BA 10® (1) | 5 | 5 |

(suite)

| Composition | Exemple 2 selon l'invention | Exemple comparatif 2 (sans charge) |
|---|---|---|
| CERAPHYL 368® (5) | 65 | 68 |
| Propyl paraben | 0,15 | 0,15 |
| Methyl paraben | 0,15 | 0,15 |
| glycérine | 10 | 10 |
| Eau permutée | 13,45 | 14,45 |
| Ethanol | 2 | 2 |
| Chlorhexidine digluconate | 0,25 | 0,25 |
| ORGASOL 2002 EXD NAT COS® (6) | 4 | |
| total | 100 | 100 |
| Total TA | 5 | 5 |
| Total huile | 65 | 68 |
| Rapport TA/Huile | 0,08 | 0,07 |
| T1 (°C) | 71 | 74 |
| T2 (°C) | 82 | 82 |
| Aspect | Crème souple, blanche, légèrement translucide | Crème souple, blanche, légèrement translucide |
| Viscosité (Pa.s) | 6,6 | 5 |
| pH | 5,9 | 5,8 |
| % démaquillage | 97 | 93 |
| Vitesse de libération de l'huile (s/100mg) | 22 | 36 |
| (1) EUMULGIN BA 10® commercialisé par Cognis = Alcool béhénylique oxyéthyléné (10 OE)<br>(5) Ethylhexyl palmitate ; CERAPHYL 368® commercialisé par ISP ou CEGESOFT C24® commercialisé par Cognis<br>(6) Nylon-12 ; ORGASOL 2002 EXD NAT COS® commercialisé par Atochem (prise d'huile : 100 ml/ 100g ; surface spécifique : 4 m2/g ; 10µ ; densité : 1,02) | | |

Il ressort de ce tableau que l'addition des particules de Nylon utilisées selon l'invention améliore nettement la vitesse de libération d'huile (ou vitesse de claquage) lors de l'application sur la peau et l'efficacité de démaquillage de la composition de l'invention.

**III. Exemples 3 à 5 comparatifs :** exemples contenant des charges n'ayant pas une prise d'huile égale ou supérieure à 75 ml/100 g.

| Composition | Exemple comparatif 3 Avec 1% de talc | Exemple comparatif 4 Avec 4 % de talc | Exemple comparatif 5 Avec 4% d'amidon |
|---|---|---|---|
| EUMULGIN BA 10® (1) | 5 | 5 | 5 |
| CERAPHYL 368® (5) | 68 | 68 | 68 |
| Propyl paraben | 0,15 | 0,15 | 0,15 |
| Methyl paraben | 0,15 | 0,15 | 0,15 |

(suite)

| Composition | Exemple comparatif 3 Avec 1% de talc | Exemple comparatif 4 Avec 4 % de talc | Exemple comparatif 5 Avec 4% d'amidon |
|---|---|---|---|
| glycérine | 10 | 10 | 10 |
| Eau permutée | 13,45 | 13,45 | 13,45 |
| Ethanol | 2 | 2 | 2 |
| Chlorhexidine digluconate | 0,25 | 0,25 | 0,25 |
| LUZENAC 15 M 00® (7) | 1 | 4 | - |
| AMIDON DE MAIS B® (8) | - | - | 4 |
| total | 100 | 100 | 100 |
| Total TA | 5 | 5 | 5 |
| Total huile | 68 | 68 | 68 |
| Rapport TA/Huile | 0,07 | 0,07 | 0,07 |
| T1 (°C) | 71 | 71 | 71 |
| T2 (°C) | 82 | 82 | 82 |
| Aspect | Crème souple, blanche, légèrement translucide | Crème souple, blanche, légèrement translucide | Crème souple, blanche, légèrement translucide |
| Viscosité (Pa.s) | 6,9 | 5,4 | 7,8 |
| pH | 5,8 | 5,8 | 5,8 |
| Vitesse de libération de l'huile (s/100mg) | 35 | 49 | 36 |

(1) EUMULGIN BA 10® commercialisé par Cognis = Alcool béhénylique oxyéthyléné (10 OE)
(5) Ethylhexyl palmitate ; CERAPHYL 368® commercialisé par ISP
(7) Talc : silicate de magnésium (CI: 77718) commercialisé par la société Luzenac (Prise d'huile: 45 ml/100g ; Granulométrie : 5$\mu$m ; densité spécifique: 2.8; densité tassée : 0.63 g/cm3 ; surface spécifique : 4.6 m$^2$/100g)
(8) ZEA MAYS (CORN) STARCH par la société Roquette Frères (prise d'huile : 58 ml/100g)

Ce tableau montre que les particules n'ayant pas les caractéristiques revendiquées ne permettent pas d'obtenir une vitesse de libération de l'huile améliorée par rapport à l'exemple comparatif 2 ne comportant pas de particules).

**IV. Exemples 3 à 6 selon l'invention**

| MP | Exemple 3 selon l'invention | Exemple 4 selon l'invention | Exemple 5 selon l'invention | Exemple 6 selon l'invention |
|---|---|---|---|---|
| EUMULGIN BA 10® (1) | 5 | 5 | 5 | 5 |
| Stéarate d'isocétyle (9) | - | - | 23.3 | - |
| CETIO LOE® (10) | - | - | 43.7 | - |
| Mineral oil (11) | 68 | - | - | 65 |
| Huile d'abricot (12) | - | 68 | 3 | - |
| Fraction liquide de beurre de karité | | | | 3 |

(suite)

| MP | Exemple 3 selon l'invention | Exemple 4 selon l'invention | Exemple 5 selon l'invention | Exemple 6 selon l'invention |
|---|---|---|---|---|
| Propyl paraben | 0,15 | 0,15 | 0,15 | 0,15 |
| Methyl paraben | 0,15 | 0,15 | 0,15 | 0,15 |
| glycérine | 10 | 10 | 10 | 10 |
| Eau permutée | Qsp 100 | Qsp 100 | Qsp 100 | Qsp 100 |
| ethanol | 2 | 2 | 2 | 2 |
| Chlorhexidine digluconate | 0,25 | 0,25 | 0,25 | 0,25 |
| AEROSIL 200® (**4**) | 1 | - | 1 | 1 |
| ORGASOL 2002 EXD NAT COS® (6) | - | 4 | - | - |
| total | 100 | 100 | 100 | 100 |
| Total TA | 5 | 5 | 5 | 5 |
| Total huile | 68 | 68 | 70 | 68 |
| Rapport TA/Huile | 0,07 | 0,07 | 0,07 | 0,07 |
| Aspect | Crème souple, blanche | Crème souple, couleur crème. | Crème souple, couleur crème. | Crème souple, couleur crème. |
| Viscosité (Pa.s) | 5,5 | 5 | 6.6 | 5 |
| pH | 5,8 | 5,8 | 5,8 | 5,8 |
| Utilisation | Crème de massage pour le corps | Crème de massage pour le corps ou le visage | Crème de massage pour le visage | Crème contour des yeux |

(1) EUMULGIN BA 10® commercialisé par Cognis = Alcool béhénylique oxyéthyléné (10 OE) (4) AEROSIL 200® commercialisé par Degussa-Hüls = Silice pyrogénée hydrophile (prise d'huile : 460 ml/100g ; Densité tassée : 50g/l ; 200 m$^2$/g)

(6) Nylon-12 ; ORGASOL 2002 EXD NAT COS® commercialisé par Atochem (prise d'huile : 100 ml/ 100g ; surface spécifique : 4 m2/g ; 10μ ; densité : 1,02)

(9) Stéarate d'isocétyle = STEARATE D'ISOCETYLE® commercialisé par Stéarineries Dubois.

(10) Di-caprylyl ether = CETIOL OE® commercialisé par Cognis (11) Mineral oil = Marcol 82® commercialisé par Esso (12) Huile d'abricot =APRICOT KERNEL OIL® commercialisé par Desert Whale

## V. Exemples 7 et 8 selon l'invention

| MP | Crème de massage exfoliante pour le corps ou le visage | Baume après-shampooing |
|---|---|---|
| EUMULGIN BA 10® (1) | 5 | 5 |
| Mineral oil (11) | - | 68 |
| CERAPHYL 368 (5) | 68 | |
| Polyquaternium-7 (13) | - | 0,5 |
| Propyl paraben | 0,15 | 0,15 |
| Methyl paraben | 0,15 | 0,15 |

(suite)

| MP | Crème de massage exfoliante pour le corps ou le visage | Baume après-shampooing |
|---|---|---|
| glycérine | 10 | 10 |
| Ethanol | 2 | 2 |
| Chlorhexidine digluconate | 0,25 | 0,25 |
| AEROSIL 200® (4) | 1 | 1 |
| GOTALENE 120 INCOLORE 2® de Dupont (14) | 4 | - |
| Eau permutée | Qsp 100 | Qsp 100 |
| total | 100 | 100 |
| Total TA | 5 | 5 |
| Total huile | 68 | 68 |
| Rapport TA/Huile | 0,07 | 0,07 |
| Aspect | Crème souple, blanche | Crème souple, couleur crème. |
| Viscosité (Pa.s) | 5,5 | 5 |
| pH | 5,8 | 5,8 |
| (1) EUMULGIN BA 10® commercialisé par Cognis = Alcool béhénylique oxyéthyléné (10 OE)<br>(4) AEROSIL 200® commercialisé par Degussa-Hüls = Silice pyrogénée hydrophile<br>(5) Ethylhexyl palmitate ; CERAPHYL 368® commercialisé par ISP<br>(13) Polyquaternium-7 = Merquat S®, commercialisé par Ondeo Nalco<br>(14) Gotalene 120 incolore 2® commercialisé par Dupont = poudre de polyéthylène | | |

**Revendications**

1. Composition pour application topique sous forme d'émulsion huile-dans-eau, **caractérisée par le fait qu'**elle comporte :

   - une phase lipophile (A), présente en une quantité d'au moins 60 % en poids par rapport au poids total de la composition,
   - une phase aqueuse (C) présente en une quantité inférieure ou égale à 30 % en poids par rapport au poids total de la composition,
   - un système émulsionnant (B) présent en une quantité de 2 à 20 % en poids par rapport au poids total de la composition et comprenant au moins un émulsionnant ayant un HLB allant de 8 à 18,
   - de 0,5 à 10 % en poids par rapport au poids total de la composition, d'une ou plusieurs charges ayant une prise d'huile égale ou supérieure à 75 ml/100 g,
   - le rapport système émulsionnant (B) / la phase lipophile (A) allant de 0,04 à 0,2,
   - le diamètre moyen en volume des gouttelettes de phase huileuse D[4,3] va de 0,09 $\mu$m à 4 $\mu$m.

2. Composition selon la revendication 1, **caractérisée par le fait qu'**elle est susceptible d'être obtenue selon la technique d'émulsification par inversion de phase.

3. Composition selon la revendication 1 ou 2, **caractérisée par le fait qu'**elle présente une viscosité mesurée à 25°C avec l'appareil de mesure Rheomat 180 à 200 rpm (tours par minute), égale ou supérieure à 1 Pa.s.

4. Composition selon l'une quelconque des revendications précédentes, **caractérisée par le fait que** l'émulsionnant est choisi parmi les alcools gras éthoxylés, les acides gras éthoxylés, les glycérides partiels d'acides gras ethoxylés, les triglycérides d'acides gras polyglycérolés et leurs dérivés éthoxylés, et leurs mélanges.

**5.** Composition selon la revendication précédente, **caractérisée par le fait que** l'émulsionnant est choisi parmi les produits d'addition d'oxyde d'éthylène avec l'alcool laurylique ; les produits d'addition d'oxyde d'éthylène avec l'alcool béhénylique ; les produits d'addition d'oxyde d'éthylène avec l'alcool cétéarylique ; les produits d'addition d'oxyde d'éthylène avec l'alcool cétylique ; les produits d'addition d'oxyde d'éthylène avec l'alcool stéarylique ; les produits d'addition d'oxyde d'éthylène avec l'alcool isostéarylique ; les produits d'addition d'oxyde d'éthylène avec les acides laurique, palmitique, stéarique ou béhénique, et leurs mélanges.

**6.** Composition selon la revendication précédente, **caractérisée par le fait que** l'émulsionnant est le beheneth-10.

**7.** Composition selon l'une quelconque des revendications précédentes, **caractérisée par le fait que** le système émulsionnant contient en outre un ou plusieurs co-émulsionnants choisis parmi les alcools gras ayant 8 à 30 atomes de carbone ; les acides gras ayant 8 à 30 atomes de carbone ; les esters gras de glycérol ; leurs dérivés oxyéthylénés comportant 2 à 8 groupes oxyde d'éthylène, et leurs mélanges.

**8.** Composition selon l'une quelconque des revendications précédentes, **caractérisée par le fait que** la quantité de système émulsionnant va de 3 à 16 % en poids par rapport au poids total de la composition.

**9.** Composition selon l'une quelconque des revendications précédentes, **caractérisée par le fait que** la quantité de phase lipophile va de 60 à 80 % en poids par rapport au poids total de la composition.

**10.** Composition selon l'une quelconque des revendications précédentes, **caractérisée par le fait que** la ou les charges sont choisies parmi les silices ayant une surface spécifique égale ou supérieure à 120 m$^2$/g, les microsphères de polyamide ayant une taille moyenne en nombre allant de 5 $\mu$m à 30 $\mu$m, et leurs mélanges.

**11.** Composition selon l'une quelconque des revendications précédente, **caractérisée par le fait que** la quantité de charges va de 0,5 à 5 % en poids par rapport au poids total de la composition.

**12.** Composition selon l'une quelconque des revendications précédentes, **caractérisée par le fait qu'**elle constitue une composition cosmétique ou dermatologique.

**13.** Composition selon l'une quelconque des revendications 1 à 11, **caractérisée par le fait qu'**elle constitue une composition de démaquillage et/ou de nettoyage de la peau, des lèvres et/ou des cils, une composition pour le massage de la peau du corps et/ou du visage, une composition exfoliante, une composition solaire et après-solaire, un baume de soin de douche, une composition après- shampooing, un baume de soin capillaire, un produit de rasage, un masque, un cataplasme amincissant, un baume de massage, un baume réparateur lèvres, un baume pour pieds secs.

**14.** Utilisation cosmétique d'une composition cosmétique selon l'une quelconque des revendications 1 à 11, pour le traitement cosmétique des matières kératiniques.

**15.** Utilisation cosmétique d'une composition cosmétique selon l'une quelconque des revendications 1 à 11, pour le démaquillage et/ou le nettoyage de la peau, des lèvres et/ou des cils.

**16.** Utilisation cosmétique d'une composition cosmétique selon l'une quelconque des revendications 1 à 11, pour le maquillage de la peau, des lèvres et/ou des cils.

**17.** Procédé de démaquillage et/ou de nettoyage de la peau, y compris du cuir chevelu, des cils et/ou des lèvres, **caractérisé par le fait que** l'on applique sur la peau, les cils et/ou les lèvres, une composition selon l'une quelconque des revendications 1 à 11.

**18.** Procédé de préparation d'une composition selon l'une quelconque des revendications 1 à 11, consistant à :

- 1) Peser dans un récipient tous les constituants de la composition (à l'exception des matières premières thermosensibles et des charges),
- 2) Homogénéiser le mélange, et chauffer en augmentant progressivement la température au moyen d'un bain marie jusqu'à une température supérieure ou égale à la température d'inversion de phase T2,
- 3) Arrêter le chauffage et maintenir l'agitation jusqu'à retour à la température ambiante, en passant par la température d'inversion de phase T1,

- 4) Lorsque la température est redescendue en dessous de la zone d'inversion de Phase en Température (T1), additionner les charges et éventuellement les matières premières thermosensibles.

**Patentansprüche**

1. Zusammensetzung zur topischen Anwendung in Form einer Öl-in-Wasser-Emulsion, **dadurch gekennzeichnet, dass** sie Folgendes aufweist:

    - eine lipophile Phase (A), die in einer Menge von mindestens 60 Gewichts-%, bezogen auf das Gesamtgewicht der Zusammensetzung, vorliegt,
    - eine wässrige Phase (C), die in einer Menge von höchstens 30 Gewichts-%, bezogen auf das Gesamtgewicht der Zusammensetzung, vorliegt,
    - ein Emulgatorsystem (B), das in einer Menge von 2 bis 20 Gewichts-%, bezogen auf das Gesamtgewicht der Zusammensetzung, vorliegt und mindestens einen Emulgator mit einem HLB von 8 bis 18 umfasst,
    - 0,5 bis 10 Gewichts-%, bezogen auf das Gesamtgewicht der Zusammensetzung, eines oder mehrerer Füllstoffe mit einem Ölaufnahmevermögen von mindestens 75 ml/100 g,
    - wobei das Verhältnis zwischen dem Emulgatorsystem (B) / der lipophilen Phase (A) im Bereich von 0,04 bis 0,2 liegt,
    - der mittlere Volumendurchmesser der Tröpfchen der Ölphase D[4,3] liegt im Bereich von 0,09 $\mu$m bis 4 $\mu$m.

2. Zusammensetzung nach Anspruch 1, **dadurch gekennzeichnet, dass** sie gemäß der Phasenumkehr-Emulsionstechnik erhalten werden kann.

3. Zusammensetzung nach Anspruch 1 oder 2, **dadurch gekennzeichnet, dass** ihre Viskosität, wenn diese bei 25 °C mittels eines Rheomat-Messgeräts mit 180 bis 200 U/min. (Umdrehungen pro Minute) gemessen wird, mindestens 1 Pa.s beträgt.

4. Zusammensetzung nach einem beliebigen der vorhergehenden Ansprüche, **dadurch gekennzeichnet, dass** der Emulgator aus den ethoxylierten Fettalkoholen, den ethoxylierten Fettsäuren, den teilweise mit Glycerin veresterten ethoxylierten Fettsäuren, den Triglyceriden von Polyglycerinfettsäuren und deren ethoxylierten Derivaten sowie Mischungen davon ausgewählt ist.

5. Zusammensetzung nach dem vorhergehenden Anspruch, **dadurch gekennzeichnet** der Emulgator aus den Additionsprodukten von Ethylenoxid und Laurylalkohol; den Additionsprodukten von Ethylenoxid und Behenylalkohol; den Additionsprodukten von Ethylenoxid und Cetylstearylalkohol; den Additionsprodukten von Ethylenoxyd und Cetylalkohol; den Additionsprodukten von Ethylenoxid und Stearylalkohol; den Additionsprodukten von Ethylenoxid und Isostearylalkohol; den Additionsprodukten von Ethylenoxid und Laurinsäure, Palmitinsäure, Stearinsäure oder Behensäure sowie Mischungen davon ausgewählt ist.

6. Zusammensetzung nach dem vorhergehenden Anspruch, **dadurch gekennzeichnet, dass** es sich bei dem Emulgator um Beneneth-10 handelt.

7. Zusammensetzung nach einem beliebigen der vorhergehenden Ansprüche, **dadurch gekennzeichnet, dass** das Emulgatorsystem darüber hinaus einen oder mehrere co-Emulgatoren enthält, die aus den Fettalkoholen mit 8 bis 30 Kohlenstoffatomen; den Fettsäuren dem 8 bis 30 Kohlenstoffatomen; den Fettsäureestern von Glycerin; deren ethoxylierten Derivaten mit 2 bis 8 Ethylenoxidgruppen sowie Mischungen davon ausgewählt ist.

8. Zusammensetzung nach einem beliebigen der vorhergehenden Ansprüche, **dadurch gekennzeichnet, dass** die Menge des Emulgatorsystems im Bereich von 3 bis 16 Gewichts-% liegt, bezogen auf das Gesamtgewicht der Zusammensetzung.

9. Zusammensetzung nach einem beliebigen der vorhergehenden Ansprüche, **dadurch gekennzeichnet, dass** die Menge der lipophilen Phase im Bereich von 60 bis 80 Gewichts-% liegt, bezogen auf das Gesamtgewicht der Zusammensetzung.

10. Zusammensetzung nach einem beliebigen der vorhergehenden Ansprüche, **dadurch gekennzeichnet, dass** die Füllstoffe aus den Siliciumdioxidarten, welche eine spezifische Oberfläche von mindestens 120 m$^2$/g aufweisen,

den Polyamid-Mikrokugeln mit einem Zahlenmittel des Größenwertes im Bereich von 5 $\mu$m bis 30 $\mu$m sowie Mischungen davon ausgewählt sind.

11. Zusammensetzung nach einem beliebigen der vorhergehenden Ansprüche, **dadurch gekennzeichnet, dass** die Menge der Füllstoffe im Bereich von 0,5 bis 5 Gewichts-% liegt, bezogen auf das Gesamtgewicht der Zusammensetzung.

12. Zusammensetzung nach einem beliebigen der vorhergehenden Ansprüche, **dadurch gekennzeichnet, dass** sie eine kosmetische oder dermatologische Zusammensetzung darstellt.

13. Zusammensetzung nach einem beliebigen der Ansprüche 1 bis 11, **dadurch gekennzeichnet, dass** sie eine Zusammensetzung zum Abschminken und/oder Reinigen der Haut, der Lippen und/oder der Wimpern, eine Zusammensetzung zum Massieren der Körper- und/oder Gesichtshaut, eine Zusammensetzung für die Mikrodermabrasion, eine Zusammensetzung für das Sonnenbaden und die Hautpflege nach der Sonneneinwirkung, einen Duschpflegebalsam, eine Haarpflegezusammensetzung zur Anwendung nach dem Shampoo, einen Haarpflegebalsam, ein Rasiermittel, eine Hautmaske, ein Pflaster mit gewichtsreduzierender Wirkung, einen Massagebalsam, einen Lippenheilbalsam, einen Balsam für trockene Füße darstellt.

14. Kosmetische Verwendung einer kosmetischen Zusammensetzung nach einem beliebigen der Ansprüche 1 bis 11, zur kosmetischen Behandlung von keratinartigen Stoffen.

15. Kosmetische Verwendung einer kosmetischen Zusammensetzung nach einem beliebigen der Ansprüche 1 bis 11, zum Abschminken und/oder Reinigen der Haut, der Lippen und/oder der Wimpern.

16. Kosmetische Verwendung einer kosmetischen Zusammensetzung nach einem beliebigen der Ansprüche 1 bis 11, zum Schminken der Haut, der Lippen und/oder der Wimpern.

17. Verfahren zum Abschminken und/oder Reinigen der Haut, einschließlich der Kopfhaut, der Wimpern und/oder der Lippen, **dadurch gekennzeichnet, dass** eine Zusammensetzung nach einem beliebigen der Ansprüche 1 bis 11 auf die Haut, die Wimpern und/oder die Lippen aufgebracht wird.

18. Verfahren zur Herstellung einer Zusammensetzung nach einem beliebigen der Ansprüche 1 bis 11, welches die folgenden Schritte umfasst:

1) Einwiegen sämtlicher Bestandteile der Zusammensetzung (mit Ausnahme der temperaturempfindlichen Ausgangsstoffe und der Füllstoffe) in einen Behälter,
2) Homogenisieren der Mischung und Erwärmen, indem die Temperatur allmählich mittels eines Heizbades bis auf eine Temperatur erhöht wird, die mindestens gleich der Phasenumkehrtemperatur T2 ist,
3) Abstellen der Heizung und Weiterrühren, bis wieder die Raumtemperatur erreicht wird, unter Durchlaufen der Phasenumkehrtemperatur T1,
4) wenn die Temperatur wieder unter den temperaturbedingten Phasenumkehrbereich (T1) abgesunken ist, Hinzufügen der Füllstoffe und gegebenenfalls der temperaturempfindlichen Ausgangsstoffe.

## Claims

1. Composition for topical application in the form of an oil-in-water emulsion, **characterized in that** it contains:

- a lipophilic phase (A) present in an amount of at least 60% by weight relative to the total weight of the composition,
- an aqueous phase (C) present in an amount of less than or equal to 30% by weight relative to the total weight of the composition,
- an emulsifying system (B) present in an amount of 2 to 20% by weight relative to the total weight of the composition and comprising at least one emulsifier having an HLB ranging from 8 to 18,
- from 0.5 to 10% by weight, relative to the total weight of the composition, of one or more fillers having an oil uptake greater than or equal to 75 ml/100 g,
- the emulsifying system (B)/lipophilic phase (A) ratio ranging from 0.04 to 0.2,
- the volume mean diameter of the droplets of oily phase D[4.3] ranges from 0.09 $\mu$m to 4 $\mu$m.

**2.** Composition according to Claim 1, **characterized in that** it can be obtained according to the phase inversion emulsification technique.

**3.** Composition according to Claim 1 or 2, **characterized in that** it has a viscosity measured at 25°C with the Rheomat 180 measuring device at 200 rpm (revolutions per minute) of greater than or equal to 1 Pa.s.

**4.** Composition according to any one of the preceding claims, **characterized in that** the emulsifier is chosen from ethoxylated fatty alcohols, ethoxylated fatty acids, partial glycerides of ethoxylated fatty acids, polyglycerolated fatty acid triglycerides and their ethoxylated derivatives, and mixtures thereof.

**5.** Composition according to preceding claim, **characterized in that** the emulsifier is chosen from the addition products of ethylene oxide with lauryl alcohol; the addition products of ethylene oxide with behenyl alcohol; the addition products of ethylene oxide with cetearyl alcohol; the addition products of ethylene oxide with cetyl alcohol; the addition products of ethylene oxide with stearyl alcohol; the addition products of ethylene oxide with isostearyl alcohol; the addition products of ethylene oxide with lauric acid, palmitic acid, stearic acid or behenic acid, and mixtures thereof.

**6.** Composition according to the preceding claim, **characterized in that** the emulsifier is beheneth-10.

**7.** Composition according to any one of the preceding claims, **characterized in that** the emulsifying system also contains one or more coemulsifier(s) chosen from fatty alcohols having 8 to 30 carbon atoms; fatty acids having 8 to 30 carbon atoms; fatty esters of glycerol; oxyethylenated derivatives thereof containing 2 to 8 ethylene oxide groups, and mixtures thereof.

**8.** Composition according to any one of the preceding claims, **characterized in that** the amount of emulsifying system ranges from 3 to 16% by weight relative to the total weight of the composition.

**9.** Composition according to any one of the preceding claims, **characterized in that** the amount of lipophilic phase ranges from 60 to 80% by weight relative to the total weight of the composition.

**10.** Composition according to any one of the preceding claims, **characterized in that** the filler (s) is (are) chosen from silicas having a specific surface area greater than or equal to 120 $m^2/g$, microspheres of polyamide having a number-average size ranging from 5 $\mu$m to 30 $\mu$m, and mixtures thereof.

**11.** Composition according to any one of the preceding claims, **characterized in that** the amount of fillers ranges from 0.5 to 5% by weight relative to the total weight of the composition.

**12.** Composition according to any one of the preceding claims, **characterized in that** it constitutes a cosmetic or dermatological composition.

**13.** Composition according to any one of Claims 1 to 11, **characterized in that** it constitutes a makeup-removing composition and/or a cleansing composition for the skin, the lips and/or the eyelashes, a composition for massaging facial skin and/or body skin, an exfoliating composition, an antisun composition or an aftersun composition, a shower care balm, a conditioner composition, a hair care balm, a shaving product, a mask, a slimming poultice, a massage balm, a lip repairing balm, a balm for dry feet.

**14.** Cosmetic use of a cosmetic composition according to any one of Claims 1 to 11, for the cosmetic treatment of keratin materials.

**15.** Cosmetic use of a cosmetic composition according to any one of Claims 1 to 11, for removing makeup from and/or for cleansing the skin, the lips and/or the eyelashes.

**16.** Cosmetic use of a cosmetic composition according to any one of Claims 1 to 11, for making up the skin, the lips and/or the eyelashes.

**17.** Process for removing makeup from and/or for cleansing the skin, including the scalp, the eyelashes and/or the lips, **characterized in that** a composition according to any one of Claims 1 to 11 is applied to the skin, the eyelashes and/or the lips.

**18.** Process for preparing a composition according to any one of Claims 1 to 11, consisting in:

- 1) Weighing out, into a container, all the constituents of the composition (with the exception of the thermosensitive starting materials and fillers).
- 2) Homogenizing the mixture, and heating by gradually increasing the temperature, by means of a water bath, to a temperature greater than or equal to the phase inversion temperature T2.
- 3) Stopping the heating and maintaining the stirring until ambient temperature is again reached, passing through the phase inversion temperature T1.
- 4) When the temperature has again dropped below the phase inversion temperature (T1) region, adding the fillers and, optionally, the thermosensitive starting materials.

## RÉFÉRENCES CITÉES DANS LA DESCRIPTION

**Documents brevets cités dans la description**

- WO 0189678 A

- JP 2295912 A

**Littérature non-brevet citée dans la description**

- **K. SHINODA.** *J. Chem. Soc. Jpn.,* 1968, vol. 89, 435
- **K. SHINODA ; H. SAITO.** *J. Colloïd Interface Sci.,* 1969, vol. 30, 258
- **MITSUI et al.** *Application of the phase-inversion-temperature method to the emulsification of cosmetics*

- **T. MITSUI ; Y. MACHIDA ; F. HARUSAWA.** *American. Cosmet. Perfum.,* 1972, vol. 87, 33
- Operators Guide. *Malvern Instruments,* Décembre 1998, 61-67